# EUROPÄISCHE PATENTSCHRIFT

(11) **EP 1 481 974 B1**
(45) Veröffentlichungstag und Bekanntmachung des Hinweises auf die Patenterteilung: **31.10.2007**
(21) Anmeldenummer: 04020713.6
(22) Anmeldetag: 21.11.2002
(51) Int. Cl.: C07D 319/06

(54) **Riechstoffe auf Basis cyclischer Acetale**
Fragrances based on cyclic acetals
Fragrances à base d acetals cycliques

(30) Priorität: 30.11.2001 DE 10158907
(43) Veröffentlichungstag der Anmeldung: 01.12.2004
(62) Teilanmeldung aus: 02025951.1
(73) Patentinhaber: OXEA Deutschland GmbH, 46147 Oberhausen (DE)
(72) Erfinder: Lappe, Peter, Dr., 46539 Dinslaken (DE); Schmid, Klaus, Dr., 46539 Dinslaken (DE); Söllner, Rolf, Dr., 46535 Dinslaken (DE); Springer, Helmut, 46539 Dinslaken (DE)

(56) Entgegenhaltungen:
- EP-A- 0 276 998
- EP-A- 0 358 091
- US-A- 3 884 841
- US-A- 4 372 880
- US-A- 4 632 515
- PATENT ABSTRACTS OF JAPAN Bd. 004, Nr. 135 (C-025), 20. September 1980 (1980-09-20) & JP 55 087733 A (MITSUBISHI PETROCHEM CO LTD), 2. Juli 1980 (1980-07-02)

## Beschreibung

Die Erfindung betrifft neue Riechstoffe auf Basis cyclischer Acetale, die sich von Diolen als Alkohol- und von substituierten Propionaldehyden als Aldehydkomponente ableiten.

In der Parfümerie wurden anfangs ausschließlich natürliche Stoffe als Geruchsträger verwendet. Diese Stoffe stehen jedoch nicht immer in ausreichender Menge und in gleichbleibender Qualität zur Verfügung. Häufig ist ihre Verwendung auch aus ökologischen Gründen nur eingeschränkt möglich und überdies sind sie oft sehr teuer, so dass sich ihr Einsatz als Geruchsgeber für Massenprodukte verbietet. Daher werden heutzutage in großem Umfang synthetische Substanzen als Riechstoffe eingesetzt.

In den letzten Jahren haben neben einer großen Anzahl anderer Verbindungsklassen cyclische Acetale und Ketale als industriell erzeugte Riechstoffe an Bedeutung gewonnen. In einer umfangreichen Arbeit in Angew. Chemie 2000, 112, 3107-3138 wird der Einsatz von cyclischen Acetalen und Ketalen als Ambra-Riechstoffe beschrieben. Beispiele für derartige Verbindungen sind u.a. Acetale und Ketale, die sich von 1-Formyl-2,4-dimethyl-cyclohexen-3 und 2-Methyl-2-sec.butyl-propan-1,3-diol und von 2-Formylcaran und Aceton ableiten. Ferner werden Dioxolane und 1,3-Dioxane als potentielle Riechstoffe erwähnt. Diese Verbindungen sind aus α-Cedren bzw. aus Campher leicht zugänglich.

So schreibt die JP 55 087 733 (Patent Abstract of Japan, Vol. 004, No. 135 (C-025) Riechstoffe auf Basis cyclischer Acetale, die aus 1,3-Dimethyl-3-cyclohexenylpropionaldehyd und einem cyclischen 1,3-Diol aufgebaut sind.

Nach US-A-4,372,880 lassen sich substituierte 1,3-Dioxane, die in 2-Position eine 1-Methylbutylgruppe tragen, in Riechstoffkompositionen einsetzen.

Cyclische Acetale auf Basis des 4,4,6-Trimethyl-1,3-dioxan mit Alkylgruppen in der 2-Position, insbesondere mit der n-Butylgruppe oder der 2-Phenylethylgruppe und ihre Verwendung als Riechstoffe sind aus US-A-3,884,841 bekannt.

EP-A2-0 358 091 behandelt cyclische Acetale auf Basis von 2-Cyclohexylpropanal und 1,3-Diolen. Dabei wird zunächst 2-Phenylpropanal mit einem Diol unter Acetalbildung umgesetzt, das dann anschließend zum Cyclohexylderivat hydriert wird. Die bekannten Verbindungen eignen sich als Zusätze in Riechstoffkompositionen.

In der EP 0 276 998 A2 werden Parfümkompositionen beschrieben, die als Riechstoff mindestens eine Verbindung der allgemeinen Formel 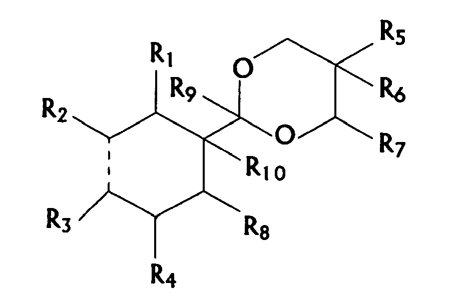 enthalten. In dieser Formel steht die gestrichelte Linie für eine Doppel- oder Einfachbindung innerhalb des Cyclohexanringes. R₁, R₂, R₃ und R₄ bedeuten Wasserstoff oder einen Methylrest. R₅ und R₆ sind Wasserstoff oder ein Alkyl-, ein Cycloalkyl- oder ein Alkenylrest mit 1 bis 4 Kohlenstoffatomen oder ein, gegebenenfalls substituierter, Ring, der bis zu 5 Kohlenstoffatome enthält. R₅ und R₆ können zusammen mit dem Kohlenstoffatom des Dioxanringes einen, gegebenenfalls substituierten, Ring mit bis zu 6 Kohlenstoffatomen bilden. R₇ bedeutet Wasserstoff, Methyl, Ethyl, n-Propyl, Isopropyl, n-Butyl, sec.Butyl, Isobutyl oder t-Butyl. R₈ und R₉ stehen jeweils für Wasserstoff oder den Methylrest. R₁₀ ist Wasserstoff, Methyl oder Isopropyl. Die Gesamtzahl der Kohlenstoffatome liegt im Bereich von 13 bis 20.

Die durch die vorstehende, allgemeine Formel beschriebenen Verbindungen decken einen weiten Riechstoffbereich ab, einige von ihnen besitzen eine überraschende Ambranote.

Die Herstellung der genannten Verbindungen erfolgt in einer ersten Stufe durch Diels-Alder-Reaktion von Dienen mit ungesättigten Aldehyden oder Ketonen, die zu cyclischen Carbonylverbindungen führt. Beispiele für geeignete Diene sind Butadien-1,3 und 2-Methyl-1,3-pentadien. Als Aldehyde oder Ketone kommen z.B. Acrolein, Methacrolein, Crotonaldehyd oder Methylvinylketon in Betracht. In einer zweiten Stufe werden die Carbonylverbindungen mit Diolen wie 2-Methyl-2-propyl-1,3-propandiol, 2-Methyl-2-sec.butyl-1,3-propandiol oder 2-Ethylhexan-1,3-diol zum erwünschten Acetal oder Ketal umgesetzt.

Es ist ebenfalls bekannt (US-A-4,632,515), dass sich Verbindungen mit einer Ethylengruppierung, die an einem C-Atom einen substituierten 1,3-Dioxanring trägt und an dem anderen C-Atom einen substiuierten Cyclohexylrest trägt, als flüssigkristalline Materialien in elektro-optischen Anwendungen eignen.

Obgleich es bereits eine Vielzahl synthetisch erzeugter Riechstoffe gibt, besteht in der industriellen Praxis weiterhin ein hoher Bedarf an Duftträgern. Das Interesse ist dabei sowohl auf die Entwicklung neuer Duftnoten gerichtet als auch auf die möglichst kostengünstige Herstellung bekannter Riechstoffe, sei es durch verfahrenstechnische Vereinfachung des Syntheseweges oder durch Auswahl preiswerter Rohstoffe. Der vorliegenden Erfindung liegt die Aufgabe zugrunde beiden Erfordernissen der industriellen Fertigung zu genügen durch Bereitstellung neuer Verbindungen mit bekannten oder neuen Duftnoten, die aus leicht zugänglichen, in ausreichenden Mengen preisgünstig zur Verfügung stehenden Ausgangsstoffen gewonnen werden können. Zusätzlich wird Wert darauf gelegt, dass der Weg zu ihrer Herstellung technisch einfach ist und keine aufwändigen oder spezialisierten Apparaturen erfordert.

Die vorliegende Erfindung betrifft Riechstoffe der allgemeinen Formel 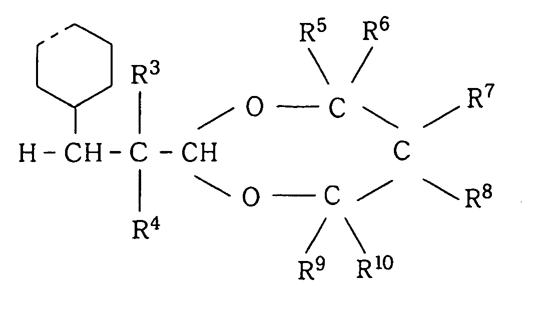 in der R⁵, R⁶, R⁷, R⁹ und R¹⁰ unabhängig voneinander jeweils Wasserstoff, einen Methylrest, einen Ethylrest oder einen n- oder i-Propylrest bedeuten, R³ und R⁴ Wasserstoff sind oder zusammen einen Methylenrest (=CH₂) bilden, R⁸ für einen geradkettigen oder verzweigten C₁- bis C₅-Alkylrest steht und die unterbrochene Linie im cyclischen Rest eine Einfach- oder Doppelbindung beschreibt.

Vorzugsweise sind R⁵, R⁷, R⁹ und R¹⁰ unabhängig voneinander jeweils Wasserstoff oder ein Methylrest.

Es wurde bereits bei der Diskussion des Standes der Technik darauf hingewiesen, daß von cyclischen aliphatischen Verbindungen, nämlich von Verbindungen des Cyclohexans und des Cyclohexens, in denen jeweils ein Ringwasserstoffatom durch eine Formylgruppe ersetzt ist, abgeleitete Acetale als Riechstoffe bekannt sind. Dennoch war es nicht zu erwarten, dass auch Acetale auf Basis ein- oder mehrfach substituierter Propionaldehyde, die als einen Substituenten eine Cyclohexyl- oder Cyclohexenylgruppe enthalten, also Formylverbindungen einer völlig anderen Struktur, Riechstoffcharakter besitzen. Hierbei ist zu berücksichtigen, dass, ähnlich wie die pharmakologische Wirksamkeit bestimmter Substanzen, auch die olfaktorischen Eigenschaften chemischer Verbindungen an ganz bestimmte Molekülstrukturen gebunden sind mit der Folge, dass bereits geringe Abweichungen von einer solchen, charakteristischen Grundstruktur, zum völligen Verlust der erwünschten und wirtschaftlich genutzten Eigenart des chemischen Stoffes führen können.

Die bei der Gewinnung der neuen Riechstoffe verwendeten Diole folgen der allgemeinen Formel 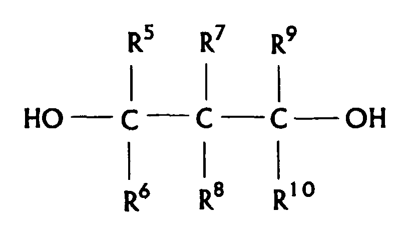 in der R⁵, R⁶, R⁷, R⁸, R⁹ und R¹⁰ die oben wiedergegebenen Bedeutungen haben. Als Beispiele für derartige zweiwertige Alkohole seien genannt: 2,2-Dimethyl-1,3-propandiol (Neopentylglykol), 2-Methyl-2-propyl-1,3-propandiol, 2-Methyl-2-sec.butyl-1,3-propandiol, 1,3-Butandiol, 2-Methyl-2,4-pentandiol, 2,2,4-Trimethyl-1,3-pentandiol, 2-Ethyl-1,3-hexandiol.

Diole der wiedergegebenen Struktur sind vielfach in technischem Maßstab verfügbar oder können nach bekannten Verfahren hergestellt werden. So erhält man 2,2-Dimethyl-1,3-propandiol durch Aldol-Addition (Aldolisierung) von Isobutyraldehyd und Formaldehyd und Hydrierung des aus dieser Umsetzung resultierenden Aldols. Unter Anwendung derselben Reaktionsstufen (d.h. Aldol-Addition und anschließende Hydrierung) gewinnt man 2-Methyl-2-propyl-1,3-propandiol aus 2-Methylpentanal und Formaldehyd und 2-Methyl-2-sec.butyl-1,3-propandiol aus 2,3-Dimethylpentanal und Formaldehyd. Die Herstellung von 2-Ethyl-1,3-hexandiol erfolgt durch Aldolisierung von n-Butyraldehyd zum 3-Hydroxy-2-ethylhexanal und nachfolgende Hydrierung. Ganz entsprechend stellt man 1,3-Butandiol durch Hydrierung des bei der Aldol-Addition von Acetaldehyd gebildeten Acetaldols, 2-Methyl-2,4-pentandiol durch Hydrierung von Diacetonalkohol, dem Aldolisierungsprodukt von Aceton und 2,2,4-Trimethyl-1,3-pentandiol durch Aldol-Addition von Isobutyraldehyd und Hydrierung des hierbei gebildeten Aldols her.

Die als weitere Komponenten zur Herstellung der beanspruchten cyclischen Acetale genannten Formylverbindungen sind ein- oder mehrfach substituierte Propionaldehyde, die als Substituenten in 3-Stellung eine Cyclohexyl- oder Cyclohexenylgruppe enthalten und der allgemeinen Formel 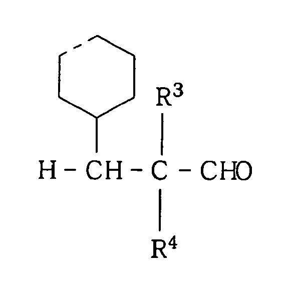 entsprechen, wobei R³ und R⁴ die weiter oben beschriebene Bedeutung haben. Beispiele für derartige Verbindungen sind 3-Cyclohexenylpropanal und 2-Methylen-3-cyclohexenylpropanal.

Die vorstehend beschriebenen Formylverbindungen werden in vielen Fällen durch Hydroformylierung (Oxosynthese) aus olefinisch ungesättigten Vorstufen gewonnen. Man ist hierbei nicht an eine bestimmte Ausführungsform der Hydroformylierungsreaktion gebunden. Daher kann man die Formylverbindung aus Olefin und Wassergas sowohl nach der klassischen Variante der Umsetzung mit Cobalt-Katalysatoren bei hohem Druck erhalten, als auch nach modernen Verfahren bei geringem Druck in Gegenwart von (gegebenenfalls modifizierten) Rhodium-Katalysatoren gewinnen.

Die Herstellung der als Riechstoffe geeigneten Acetale erfolgt in bekannter Weise durch Umsetzung von Aldehyd und Diol in Gegenwart eines sauren Katalysators [vgl. z.B. Houben-Weyl, Methoden der organischen Chemie, Band E3 Seiten 615ff, (1983)] Das molare Verhältnis der Reaktionspartner kann in weiten Bereichen variieren und beträgt üblicherweise (Aldehyd : Diol) 1:0,2 bis 1:5. Bevorzugt wendet man die Ausgangsstoffe im Molverhältnis 1:1,2 bis 1:0,8 und insbesondere 1:1 an. Als Katalysatoren werden ausschließlich saure Verbindungen eingesetzt, insbesondere technisch leicht verfügbare Säuren wie Schwefelsäure, p-Toluolsulfonsäure, Methansulfonsäure und Chlorwasserstoff. In Sonderfällen finden als Katalysatoren auch andere, sauer reagierende Verbindungen Anwendung, z.B. Ammoniumchlorid, Calciumchlorid, Zinkchlorid, Natriumhydrogensulfat und insbesondere Eisen(III)-chlorid (vgl. Houben-Weyl, I.c., Band VII, Teil 1, Seiten 417ff (1954)). Die Katalysatoren gelangen üblicherweise in Mengen von weniger als 1 Gew.-%, bezogen auf den Aldehyd, zum Einsatz. Die bevorzugt verwendeten Säuren wie Schwefelsäure und p-Toluolsulfonsäure werden in Mengen von 1 bis 50 mmol, insbesondere 1 bis 10 mmol je mol Aldehyd eingesetzt. Die Reaktionszeit, bestimmt durch Messung der abgeschiedenen Wassermenge (die theoretisch 1 mol Wasser je mol Acetal entspricht), beträgt im allgemeinen 1 bis 10 Stunden, in den meisten Fällen ist die Reaktion nach 1 bis 3 Stunden beendet. Die Reaktionstemperatur ist abhängig vom eingesetzten Aldehyd und des zur Entfernung des Wassers verwendeten Azeotropbildners (Schleppmittels). Als Schleppmittel haben sich insbesondere Kohlenwasserstoffe wie Cyclohexan, Toluol und Hexen-1 bewährt. In der Regel arbeitet man bei Normaldruck, die Anwendung geringen Unterdrucks (z.B. 500 hPa) ist jedoch nicht ausgeschlossen. Zur Aufarbeitung wird das Reaktionsgemisch gewöhnlich auf 20 bis 50°C abgekühlt und mit verdünnter wäßriger Alkalilösung z.B. Natronlauge, bis zur Einstellung eines pH-Wertes von etwa 6,5 bis 7,5, vorzugsweise 7,0, versetzt. Darauf trennt man wäßrige und organische Phase voneinander, wäscht die organische Phase mit Wasser und trennt erneut die Phasen. Anschließend reinigt man die organische Phase durch Destillation in konventionellen Apparaturen. Die Destillation kann bei Normaldruck erfolgen, wegen der hohen Siedepunkte der Acetale bevorzugt man jedoch verminderten Druck. Die Reinheit der so gewonnen Acetale liegt nach GC-Analyse im allgemeinen oberhalb 98%.

Die erfindungsgemäßen Acetale zeichnen sich durch einen angenehmen leicht erdigen Geruch aus. Sie besitzen hohe Haftfestigkeit. Physiologisch Unverträglichkeiten, d.h. Schädigungen am oder im Körper wurden nicht beobachtet. Überdies sind sie beständig gegenüber Licht, Luft und das umgebende Medium. Sie können allein in unverdünnter Form oder auf Grund ihrer Löslichkeit oder Homogenisierbarkeit mit weiteren Komponenten verdünnt und gegebenenfalls zusammen mit üblichen Hilfsstoffen wie Fixateuren, angewandt werden. Mit anderen Riechstoffen in verschiedenen Mengenverhältnissen gemischt, ergeben sie neue Riechstoffkompositionen. Im allgemeinen beträgt ihr Anteil in derartigen Kompositionen 0,5 bis 5 Gew.-%, bezogen auf die gesamte Komposition. Die erfindungsgemäßen Riechstoffe und aus ihnen hergestellte Kompositionen können unmittelbar als Parfüm dienen oder auch zur Parfümierung von Kosmetika wie Cremes, Lotionen, Duftwässern, Aerosolen, Seifen, verwendet werden. Mit gleich gutem Erfolg können sie auch zur Geruchsverbesserung von technischen Produkten wie Wasch- und Reinigungsmitteln eingesetzt werden. Zur Parfümierung der verschiedenen Produkte werden die neuen Riechstoffe oder mit ihnen hergestellte Kompositionen in Konzentrationen von 0,05 bis 2 Gew.-%, bezogen auf das Produkt-/ Riechstoffgemisch, angewandt.

In den nachfolgenden Beispielen wird ein Weg zur Herstellung der neuen Verbindungen näher beschrieben, selbstverständlich ist die Gewinnung der Acetale nicht auf den wiedergegebenen Syntheseweg beschränkt. Die in Klammern beigefügten Ziffern VIII und IX sowie X bis XVII weisen auf die Formelbilder im Anschluss an die Beispiele hin.

### Beispiel 8

### Herstellung von 2-[2-(3-Cyclohexen-1-yl)-ethyl]-5-methyl-5-(1-methylpropyl)-1,3-dioxan (VIII)

In einem mit Rührer, Wasserabscheider und Thermometer ausgestatteten 4 I-Dreihalskolben werden 691,0 g (5,0 mol) 3-Cyclohexenylpropanal und 731,0 g (5 mol) 2-Methyl-2-sec.butyl-1,3-propandiol in Gegenwart von 1,9 g (10 mmol) p-Toluolsulfonsäure und 460 g Cyclohexan umgesetzt. Nach destillativer Aufarbeitung des Reaktionsgemisches erhält man 1 227 g des Acetals. Es siedet bei 5 mbar (500 Pa) im Bereich von 155 bis 159°C, seine Reinheit beträgt 98,1%, die Ausbeute 92,1%.

### Beispiel 9

### Herstellung von 2-[2-(3-Cyclohexen-1-yl)-1-methylenethyl]-5-methyl-5-(1-methylpropyl)-1,3-dioxan (IX)

Analog Beispiel 8 werden 751,1 g (5 mol) 2-Methylen-3-cyclohexenylpropanal und 731,0 g (5 mol) MsBPD in Gegenwart von 1,9 g (10 mmol) p-Toluol-sulfonsäure und 450 g Cyclohexan umgesetzt. Nach destillativer Aufarbeitung des Reaktionsgemisches erhält man 1 235,5 g des Acetals. Es siedet bei 5 mbar (500 Pa) im Bereich von 154 bis 160°C, seine Reinheit beträgt 98,1%, die Ausbeute 88,7%.

### Beispiel 10

### Herstellung von 2-[2-(3-Cyclohexen-1-yl)-ethyl]-5,5-dimethyl-1,3-dioxan (X)

In einem 500 ml-Dreihalskolben mit Wasserabscheider, Rührer und Thermometer wird eine Lösung von 110,5 g (0,80 mol) 3-(3-Cyclohexenyl)propanal (98%) in 59,4 g Cyclohexan mit 87,5 g (0,84 mol) Neopentylglykol und 0,38 g (2,0 mmol) p-Toluolsulfonsäure unter Rückfluß bis zur Beendigung der Wasserabscheidung erhitzt. Nach Abkühlen wird das farblose Rohprodukt über eine 30 cm-Vigreux-Kolonne zunächst bei einem Druck von 250 hPa zur Entfernung der Leichtsieder destilliert. Die weitere Destillation bei 7-8 hPa liefert 164,3 g des Acetals in einer Reinheit von 97,1% (Siedebereich bei einem Druck von 7-8 hPa: 98-102°C, Ausbeute: 93,4% d. Th.).

### Beispiel 11

### Herstellung von 2-[2-(3-Cyclohexen-1-yl)-ethyl]-5-methyl-5-propyl-1,3-dioxan (XI)

Analog Beispiel 10 werden 110,5 g (0,80 mol) 3-(3-Cyclohexenyl)propanal (98%) in 66,5 g Cyclohexan mit 111,1 g (0,84 mol) 2-Methyl-2-propyl-1,3-propandiol und 0,38 g (2,0 mmol) p-Toluolsulfonsäure umgesetzt. Nach Abkühlen der Reaktionsmischung wird diese mit destilliertem Wasser neutral gewaschen. Die destillative Aufarbeitung der organischen Phase liefert 183,5 g des Acetals in einer Reinheit von 98,6% (Siedebereich bei einem Druck von 6 hPa: 122-125°C, Ausbeute: 92,8% d. Th.).

### Beispiel 12

### Herstellung von 2-[2-(3-Cyclohexen-1-yl)-ethyl]-4-isopropyl-5,5-dimethyl-1,3-dioxan (XII)

Analog Beispiel 10 werden 113,8 g (0,80 mol) 3-(3-Cyclohexenyl)propanal (98%) in 71,7 g Cyclohexan mit 125,3 g (0,84 mol) 2,2,4-Trimethyl-1,3-pentandiol und 0,38 g (2,0 mmol) p-Toluolsulfonsäure umgesetzt. Nach Abkühlen der Reaktionsmischung wird diese mit destilliertem Wasser neutral gewaschen. Die destillative Aufarbeitung der organischen Phase liefert 195,6 g des Acetals in einer Reinheit von 95,9% (Siedebereich bei einem Druck von 5 hPa: 137-138°C, Ausbeute: 91,8% d. Th.).

### Beispiel 13

### Herstellung von 2-[2-(3-Cyclohexen-1-yl)-ethyl]-4-propyl-5-ethyl-1,3-dioxan (XIII)

Analog Beispiel 10 werden 110,5 g (0,80 mol) 3-(3-Cyclohexenyl)propanal (98%) in 68,3 g Cyclohexan mit 122,8 g (0,84 mol) 2-Ethyl-1,3-hexandiol und 0,38 g (2,0 mmol) p-Toluol-sulfonsäure umgesetzt. Nach Abkühlen der Reaktionsmischung wird diese mit destilliertem Wasser neutral gewaschen. Die destillative Aufarbeitung der organischen Phase liefert 192,1 g des Acetals in einer Reinheit von 99,8 % (Siedebereich bei einem Druck von 6 hPa: 126-131°C, Ausbeute: 92,0 % d. Th.).

### Beispiel 14

### Herstellung von 2-[2-(3-Cyclohexen-1-yl)-1-methylen-ethyl]-5,5-dimethyl-1,3-dioxan (XIV)

Analog Beispiel 10 werden 123,2 g (0,80 mol) 2-Methylen-3-(3-cyclohexenyl)propanal (97,5%) in 63,5 g Cyclohexan mit 88,4 g (0,84 mol) Neopentylglykol und 0,60 g (3,2 mmol) p-Toluolsulfonsäure umgesetzt. Die destillative Aufarbeitung der organischen Phase liefert 168,2 g des Acetals in einer Reinheit von 97,5% (Siedebereich bei einem Druck von 5 hPa: 126-128°C, Ausbeute: 89,0 % d. Th.).

### Beispiel 15

### Herstellung von 2-[2-(3-Cyclohexen-1-yl)-1-methylen-ethyl]-5-methyl-5-propyl-1,3-dioxan (XV)

Analog Beispiel 10 werden 123,2 g (0,80 mol) 2-Methylen-3-(3-cyclohexenyl)propanal (97,5%) in 70,3 g Cyclohexan mit 111,1 g (0,84 mol) 2-Methyl-2-propyl-1,3-propandiol und 0,60 g (3,2 mmol) p-Toluolsulfonsäure umgesetzt. Nach Abkühlen der Reaktionsmischung wird diese mit destilliertem Wasser neutral gewaschen. Die destillative Aufarbeitung der organischen Phase liefert 193,4 g des Acetals in einer Reinheit von 97,5% (Siedebereich bei einem Druck von 5 hPa: 146-152°C, Ausbeute: 91,5 % d. Th.).

### Beispiel 16

### Herstellung von 2-[2-(3-Cyclohexen-1-yl)-1-methylen-ethyl]-4-isopropyl-5,5-dimethyl-1,3-dioxan (XVI)

Analog Beispiel 10 werden 123,2 g (0,80 mol) 2-Methylen-3-(3-cyclohexenyl)propanal (97,5%) in 74,6 g Cyclohexan mit 125,3 g (0,84 mol) 2,2,4-Trimethyl-1,3-pentandiol und 0,60 g (3,2 mmol) p-Toluolsulfonsäure umgesetzt. Nach Abkühlen der Reaktionsmischung wird diese mit destilliertem Wasser neutral gewaschen. Die destillative Aufarbeitung der organischen Phase liefert 202,9 g des Acetals in einer Reinheit von 96,0% (Siedebereich bei einem Druck von 5 hPa: 144-148 °C, Ausbeute: 91,1 % d. Th.).

### Beispiel 17

### Herstellung von 2-[2-(3-Cyclohexen-1-yl)-1-methylen-ethyl]-4-propyl-5-ethyl-1,3-dioxan (XVII)

Analog Beispiel 10 werden 123,2 g (0,80 mol) 2-Methylen-3-(3-cyclohexenyl)propanal (97,5%) in 73,8 g Cyclohexan mit 122,8 g (0,84 mol) 2-Ethyl-1,3-hexandiol und 0,60 g (3,2 mmol) p-Toluolsulfonsäure umgesetzt. Nach Abkühlen der Reaktionsmischung wird diese mit destilliertem Wasser neutral gewaschen. Die destillative Aufarbeitung der organischen Phase liefert 203,6 g des Acetals in einer Reinheit von 92,6% (Siedebereich bei einem Druck von 5 mbar: 151-154 °C, Ausbeute: 95,6 % d. Th.).

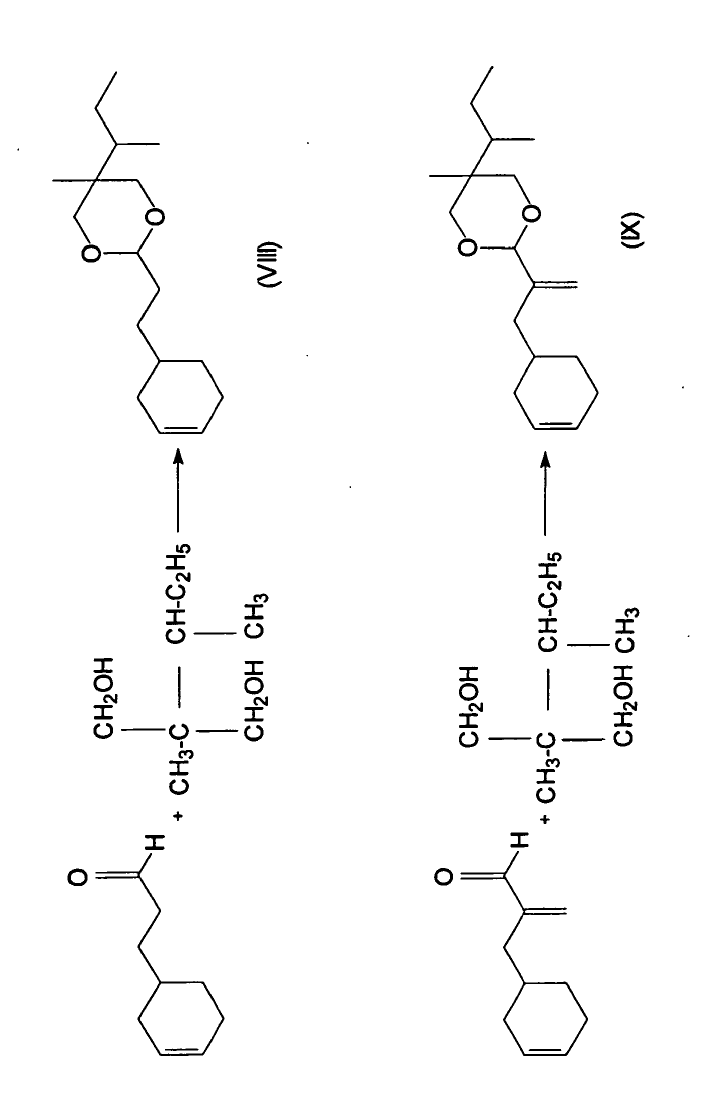

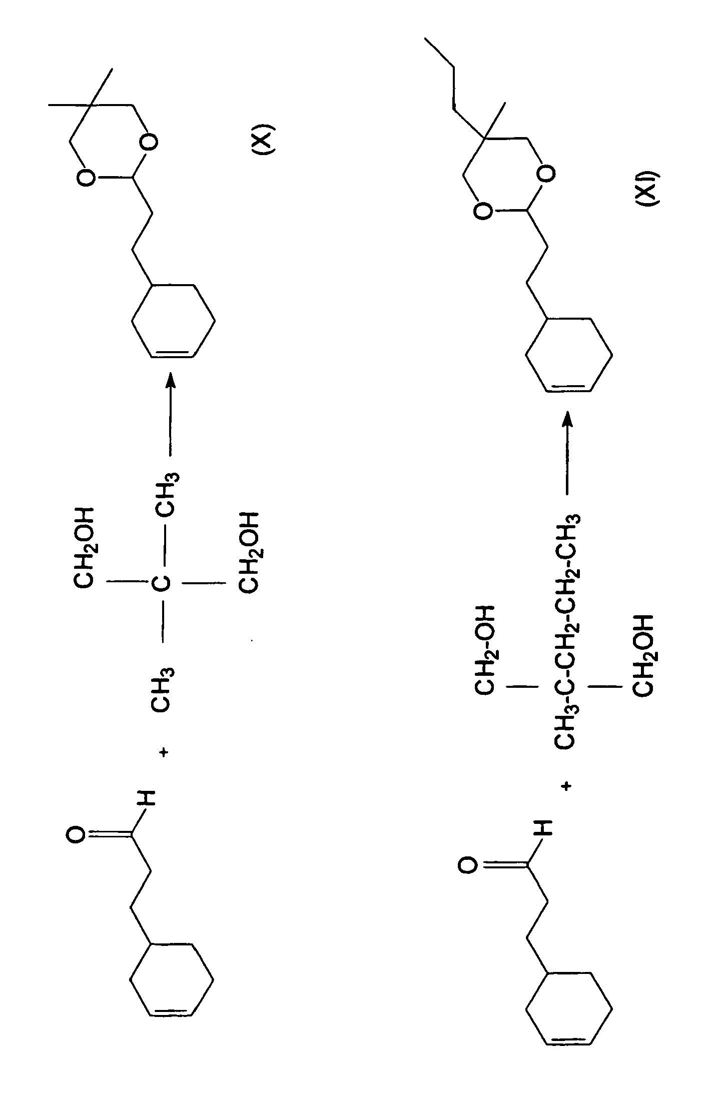

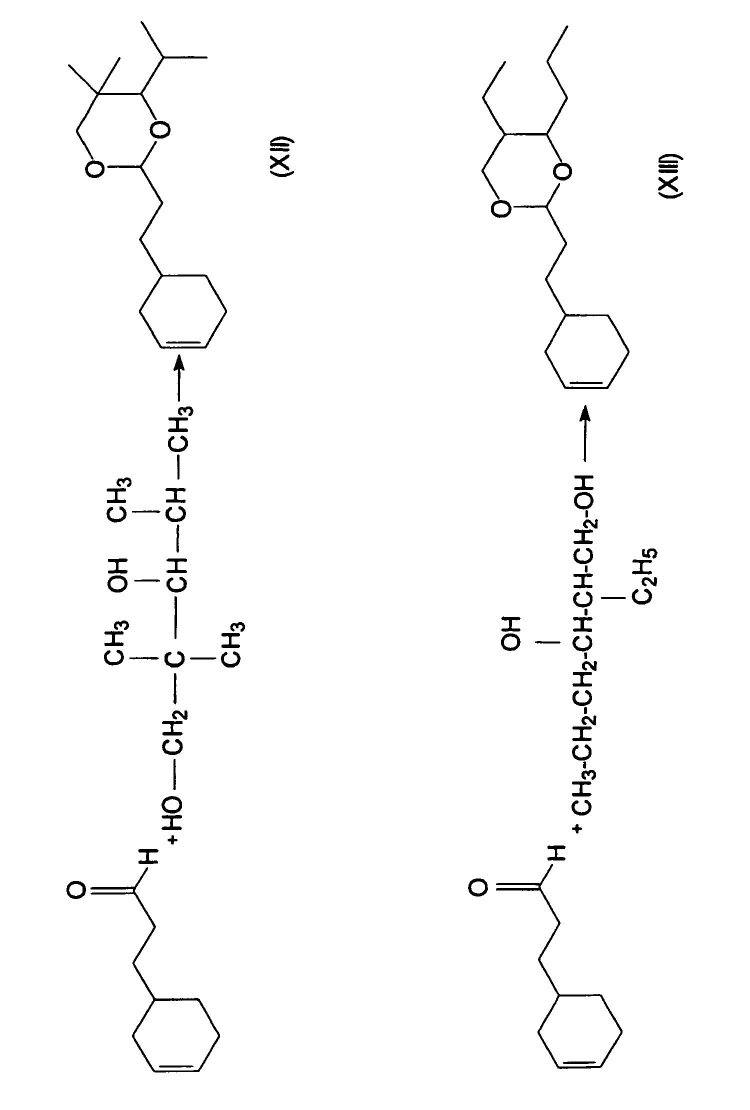

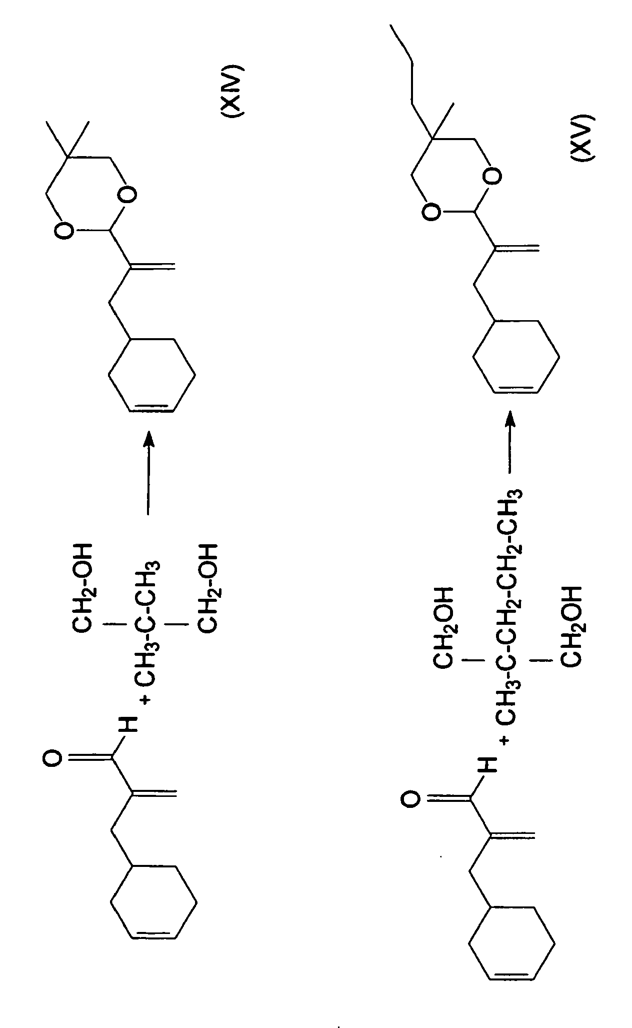

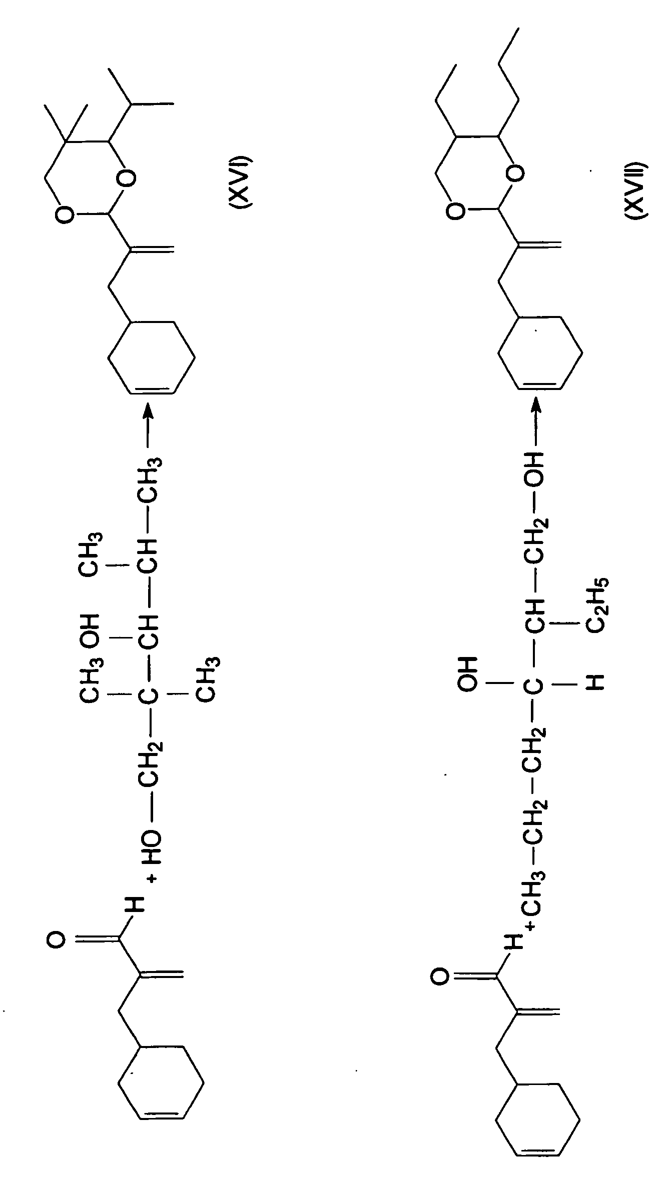

## Patentansprüche

1. Riechstoffe der allgemeinen Formel 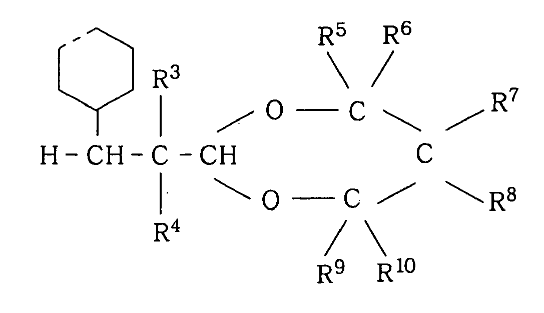 in der R⁵, R⁶, R⁷, R⁹ und R¹⁰ unabhängig voneinander jeweils Wasserstoff, einen Methylrest, einen Ethylrest oder einen n- oder i-Propylrest bedeuten, R³ und R⁴ Wasserstoff sind oder zusammen einen Methylenrest (=CH₂) bilden, R⁸ für einen geradkettigen oder verzweigten C₁- bis C₅-Alkylrest steht und die unterbrochenen Linie im cyclischen Rest eine Einfach- oder Doppelbindung beschreibt.

2. Riechstoffe nach Anspruch 1 **dadurch gekennzeichnet, dass** R⁵, R⁷, R⁹ und R¹⁰ unabhängig voneinander jeweils Wasserstoff oder ein Methylrest sind.

3. Verfahren zur Herstellung von Riechstoffen nach den Ansprüchen 1 oder 2 **dadurch gekennzeichnet, dass** man einen Aldehyd der allgemeinen Formel 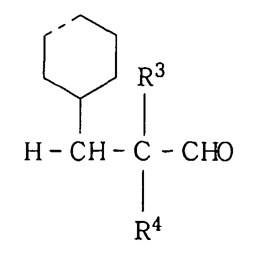 in der R³ und R⁴ Wasserstoff sind oder zusammen einen Methylenrest bilden, und die unterbrochene Linie im cyclischen Rest eine Einfach- oder eine Doppelbindung beschreibt, mit einem Diol der allgemeinen Formel 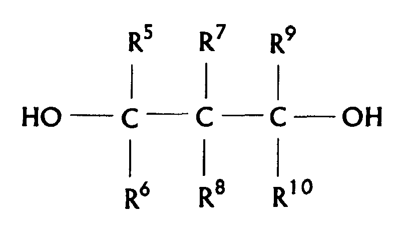 in der R⁵, R⁶, R⁷, R⁹ und R¹⁰ unabhängig voneinander jeweils Wasserstoff, einen Methylrest, einen Ethylrest oder einen n- oder i-Propylrest bedeuten und R⁸ für einen geradkettigen oder verzweigten C₁- bis C₅-Alkylrest steht, im Molverhältnis 1:0,2 bis 1:5 in Gegenwart eines saueren Katalysators und eines Schleppmittels zur Entfernung des bei der Reaktion entstehenden Wassers bei erhöhter Temperatur umsetzt, das Reaktionsgemisch auf 20 bis 50°C abkühlt, darauf verdünnte wässriger Alkalilösung bis zur Einstellung eines pH-Wertes von etwa 6,5 bis 7,5 zufügt, anschließend wässrige und organische Phase voneinander trennt, die organische Phase mit Wasser wäscht, erneut von der wässrigen Phase trennt und durch Destillation reinigt.

4. Die chemische Verbindung 2-[2-(3-Cyclohexen-1-yl)-ethyl]-5-methyl-5-(1-methylpropyl)-1,3-dioxan.

5. Die chemische Verbindung 2-[2-(3-Cyclohexen-1-yl)-1-methylenethyl]-5-methyl-5-(1-methylpropyl)-1,3-dioxan.

6. Riechstoffkompositionen enthaltend Riechstoffe nach einem oder mehreren der Ansprüche 1 und 2 und 4 und 5.

## Claims

1. A fragrance of the formula 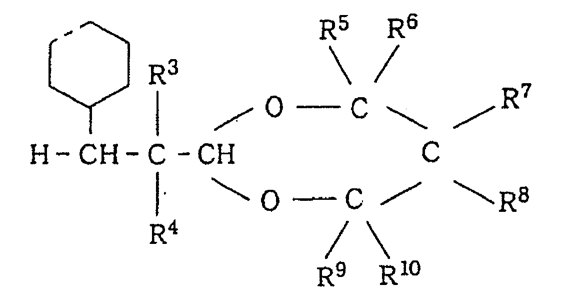 in which R⁵, R⁶, R⁷, R⁹ and R¹⁰, independently of one another, are in each case hydrogen, a methyl radical, an ethyl radical or an n- or isopropyl radical, R³ and R⁴ are hydrogen or together form a methylene radical (=CH₂), R⁸ is a straight-chain or branched C₁- to C₅-alkyl radical, and the broken line in the cyclic radical describes a single or double bond.

2. The fragrance as claimed in claim 1, wherein R⁵, R⁷, R⁹ and R¹⁰, independently of one another, are in each case hydrogen or a methyl radical.

3. A method of preparing fragrances as claimed in claims 1 or 2, which comprises reacting an aldehyde of the formula 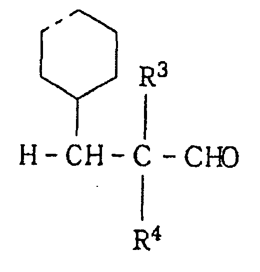 in which R³ and R⁴ are hydrogen or together form a methylene radical, and the broken line in the cyclic radical describes a single or a double bond, with a diol of the formula 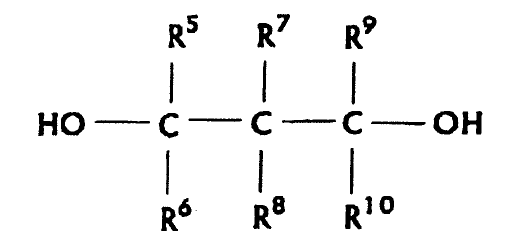 in which R⁵, R⁶, R⁷, R⁹ and R¹⁰, independently of one another, are in each case hydrogen, a methyl radical, an ethyl radical or an n- or isopropyl radical, and R⁸ is a straight-chain or branched C₁- to C₅-alkyl radical, in the molar ratio 1:0.2 to 1:5 in the presence of an acidic catalyst and an entrainer for removing the water which forms during the reaction at elevated temperature, cooling the reaction mixture to 20 to 50°C, adding dilute aqueous alkali metal solution to establish a pH of from about 6.5 to 7.5, then separating aqueous and organic phase from one another, washing the organic phase with water, separating it again from the aqueous phase and purifying it by distillation.

4. The chemical compound 2-[2-(3-cyclohexen-1-yl)-ethyl]-5-methyl-5-(1-methylpropyl)-1,3-dioxane.

5. The chemical compound 2-[2-(3-cycohexen-1-yl)-1-methyleneethyl]-5-methyl-5-(1-methylpropyl)-1,3-dioxane.

6. A fragrance composition comprising fragrances as claimed in one or more of claims 1 and 2 and 4 and 5.

## Revendications

1. Parfums de formule générale 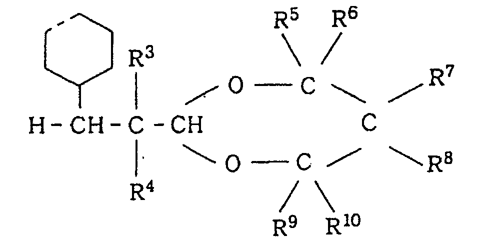 dans laquelle R⁵, R⁶, R⁷, R⁹ et R¹⁰ représentent chacun, indépendamment les uns des autres, un atome d'hydrogène, un radical méthyle, un radical éthyle ou un radical n- ou isopropyle, R³ et R⁴ sont un atome d'hydrogène ou forment conjointement un radical méthylène (=CH₂), R⁸ représente un radical alkyle en C₁-C₅ à chaîne droite ou ramifiée et le trait interrompu dans le radical cyclique signifie une liaison simple ou une liaison double.

2. Parfums selon la revendication 1, **caractérisés en ce que** R⁵, R⁷, R⁹ et R¹⁰ représentent chacun, indépendamment les uns des autres, un atome d'hydrogène ou un radical méthyle.

3. Procédé pour la préparation de parfums selon la revendication 1 ou 2, **caractérisé en ce qu'**on fait réagir à une température élevée un aldéhyde de formule générale 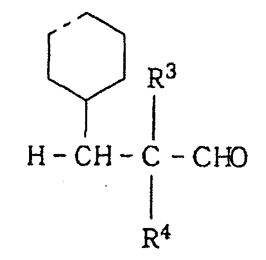 dans laquelle R³ et R⁴ sont un atome d'hydrogène ou forment conjointement un radical méthylène et le trait interrompu dans le radical cyclique signifie une liaison simple ou une liaison double, avec un diol de formule générale 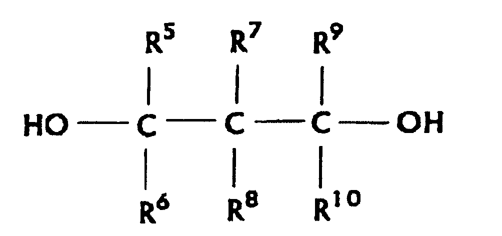 dans laquelle R⁵, R⁶, R⁷, R⁹ et R¹⁰ représentent chacun, indépendamment les uns des autres, un radical méthyle, un radical éthyle ou un radical n- ou isopropyle et R⁸ représente un radical alkyle en C₁-C₅ à chaîne droite ou ramifiée, en un rapport molaire de 1:0,2 à 1:5, en présence d'un catalyseur acide et d'un agent d'entraînement pour l'élimination de l'eau formée au cours de la réaction, on refroidit le mélange réactionnel jusqu'à 20-50°C, puis on y ajoute une solution aqueuse alcaline diluée jusqu'à l'ajustement d'un pH d'environ 6,5 à 7,5, on sépare ensuite la phase aqueuse et la phase organique, on lave avec de l'eau la phase organique, on la sépare à nouveau de la phase aqueuse et on la purifie par distillation.

4. Le composé chimique 2-[2-(3-cyclohexén-1-yl)éthyl]-5-méthyl-5-(1-méthylpropyl)-1,3-dioxanne.

5. Le composé chimique 2-[2-(3-cyclohexén-1-yl)-1-méthylèneéthyl]-5-méthyl-5-(1-méthylpropyl)-1,3-dioxanne.

6. Compositions de parfums contenant des parfums selon une ou plusieurs des revendications 1 et 2 et 4 et 5.
